# EUROPEAN PATENT APPLICATION

(11) **EP 2 600 155 A1**
(43) Date of publication of application: **05.06.2013**
(21) Application number: 12172376.1
(22) Date of filing: 18.06.2012
(51) Int. Cl.: G01N 33/68

(54) **sFlt-1 based diagnosis and monitoring of stroke patients**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Horsch, Andrea, Luzern (CH); Zdunek, Dietmar, 82327 Tutzing (DE)
(74) Representative: Dick, Alexander

(57) **Abstract**

Disclosed is a method for diagnosing chronic cardiac ischemia in a subject suffering from ischemic stroke. The method is based on the determination of soluble fms-like tyrosine kinase-1 (sFlt-1) in a sample from the subject. Further disclosed is a method for predicting in a subject suffering from ischemic stroke the risk to suffer from infarction of the ischemic penumbra as well as a method for deciding whether a subject who suffers from ischemic stroke is susceptible to a therapy that lowers to blood pressure and/or to a therapy that increases cerebral perfusion. These methods are also based on the determination sFlt-1 in a sample from the subject.

## Description

Disclosed is a method for diagnosing chronic cardiac ischemia in a subject suffering from ischemic stroke. The method is based on the determination of soluble fms-like tyrosine kinase-1 (sFlt-1) in a sample from the subject. Further disclosed is a method for predicting in a subject suffering from ischemic stroke the risk to suffer from infarction of the ischemic penumbra as well as a method for deciding whether a subject who suffers from ischemic stroke is susceptible to a therapy that lowers to blood pressure and/or to a therapy that increases cerebral perfusion. Said methods are also based on the determination sFlt-1 in a sample from the subject. Also disclosed are kits and devices adapted to carry out the said methods.

Stroke ranks after ischemic heart disease second as a cause of lost disability-adjusted life-years in high-income countries and as a cause of death worldwide. Acute stroke is caused by a cascade of events from energy depletion to cell death after arterial occlusion. A central core of very low perfusion is surrounded by an area of dysfunction caused by metabolic and ionic disturbances, but in which structural integrity is preserved. In case reperfusion of the ischemic area is not achieved, irreversible damage will occur (Dirnagel U. et al, Trends in Neuroscience 1999: 22: 391 - 7).

Clinically acute ischemic stroke is typically characterized by the sudden onset of a focal neurologic deficit, common deficits include dysphasia, dysarthria, hemianopia, weakness, ataxia, sensory loss, and neglect. Symptoms and signs are unilateral and consciousness is in general normal or slightly impaired. Ischemic stroke may be mimicked by migraine, postictal paresis, hypoglycaemia, conversion disorder, subdural hematoma or brain tumors. In patients with transitory ischemic stroke, symptoms may be temporary and may have disappeared as the patient is seen by the physician which creates diagnostic difficulties for the attending physician (van der Worp B and van Gijn J. et al, NEJM 2007: 357: 572 - 578).

Causes of stroke are variable and include large vessel atherosclerosis (with reduced blood flow or as a source of embolization), cardioembolic stroke (frequently associated thrombus formation in the left atria) or lacunar or small vessel stroke. Depending on the extent and duration of reduced blood flow TIA (without cerebral necrosis) or minor (nor disabling) or major (disabling) stroke may develop.

Current treatment of stroke targets early revascularisation, rehabilitation, and prevention of recurrent stroke. There are however no concepts as to treat underlying heart disease in these patients specifically.

A fall in cerebral blood flow to zero causes death of brain tissue within 4 - 10 minutes, values below 16 - 18 mL (100 g tissue per min cause infarction with in an hour, and values below 20mL/100 g tissue per minute cause ischemia without infarction unless prolonged for several hours or days. Tissue surrounding the core region of infarction is ischemic but reversibly dysfunctional, referred to as "ischemic penumbra". Whereas the ischemic penumbra is viable, the core region is not viable anymore. The ischemic penumbra will eventually infarct if no change in flow occurs, and hence saving the ischemic penumbra is the goal of revascularisation therapies (Smith W.S. et al. in Harrison, Principles of Internal Medicine p 2513 ff 17. Edition). Therefore, it is of particular importance to ensure that the reversibly dysfunctional regions of the brain is supplied with sufficient oxygen. This can be e.g. achieved by increasing cardiac perfusion.

Moreover, elevated blood pressure is often detected in the first hours after stroke. E.g. Robinson et al. (The predictive role of 24-hour compared to causal blood pressure levels on outcome following acute stroke. Cerebrovasc Dis. 1997; 7: 264-272) describes that blood pressure of larger than 160 mm Hg are detected in more 60% of patients with acute stroke. High blood pressure usually is an indicator for poor outcome. Therefore, a therapy that aims to lower the blood pressure is frequently initiated after the diagnosis of stroke. There is significant uncertainty about lowering blood pressure in stroke patients. Aggressive treatment of blood pressure may lead to neurological worsening by reducing perfusion pressure to ischemic areas of the brain. Therefore, lowering blood pressure may further compromise already ischemic brain, potentially increasing the size of the stroke. If treatment is necessary, precipitous drops should be avoided.

Another important aspect of stroke is interaction with preexisting cardiac disease. In a large study in stroke patient with pre-existent cardiac disease ECG abnormalities were frequent and were found in 55.3 % of patients (Christensen H. et al J Neurolog Sciece 2005: 234: 99 - 103). Even more importantly ST segment changes resulting from the stroke were also found (Christensen H et al.), also changes in heart rate could be found (with a significant decline over time). This is further evidence that ischemic stroke has impact on cardiac function. This is further supported by the observation that heart failure patients with low output may suffer from increased vulnerability of the brain by hypoperfusion and a decreased global (cerebral) blood flow. This fact and the presence of altered cerebrovascular reactivity makes these patients also vulnerable to hypotension (Pullicino P.M. et al, Stroke 2009: 40: 3706 - 3710). The need to retain systolic blood pressure in stroke is however not consistent with heart failure therapy recommendations to maintain systolic blood pressure at the lowest tolerable level which is achieved with ACE inhibitors, Beta adrenergic blockers and diuretics (Pullicino P.M. et al). Thus, there is a therapeutic dilemma with currently no diagnostic methods available.

In summary, diagnosis of chronic cardiac ischemia in stroke patients is of importance as in addition to the occlusion or near occlusion of a cerebral vessel, reduced blood cerebral blood flow requires consideration which can contribute to worsening of stroke. Patients with cardiac ischemia therefore require therapy of the cardiac disease in addition to standard stroke treatment.

Soluble Flt-1 (sFlt-1, soluble fms-like tyrosine kinase-1, frequently also referred to as VEGF receptor-1) is a splice variant of VEGF receptor 1 which is produced by a variety of tissues. sFlt-1 binds the proangiogenic factors vascular endothelial growth factor (VEGF) and placental growth factor (PlGF). sFlt-1 is used - in combination with PlGF - as a marker for discriminating between uncomplicated pregnancy and pregnancy with preeclampsia (Troisi et al. (2008) Am J Obstet Gynecol 199(6)).

Very recently it has been learned that acute cardiovascular events such as acute coronary syndrome with or without myocardial infarction are associated with sharp increases of sFlt-1 shortly after the ischemic event (WO 2010/144553 A 2). Moreover, US 2007/0218498, suggests sFlt-1 as a marker of ischemic stroke.

It is desirable to diagnose cardiac ischemia in patients with ischemic stroke as it contributes to (preventable) progression of stroke, and, thus, severity. The identification of stroke patients suffering from cardiac ischemia is important since these patients are at risk of suf fering from infarction of the ischemic penumbra. Therefore, treatment regimens have to be initiated in these patients which aim to increase cardiac perfusion, and, thus, may allow for preventing further damage to the brain, i.e. infarction of the ischemic penumbra.

The technical problem underlying the present invention can be seen as the provision of means and methods which comply with the aforementioned needs. The technical problem is solved by the embodiments characterized in the claims and herein below.

Accordingly, to the present invention relates to a method for diagnosing chronic cardiac ischemia in a subject suffering from ischemic stroke, said method comprising the steps of
a) determining the amount of soluble fms-like tyrosine kinase-1 (sFlt-1) in a sample from said subject, and
b) comparing the amount of sFlt-1 in said sample to a reference amount, whereby chronic cardiac ischemia is diagnosed.

Preferably, chronic cardiac ischemia is diagnosed by carrying out the further step of c) diagnosing chronic cardiac ischemia in said subject based on the result of the comparison carried out in step b).

The method of the present invention, preferably, is an ex vivo or in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "diagnosing" as used herein, preferably, means assessing whether a subject suffers from chronic cardiac ischemia or not. As will be understood by those skilled in the art, such an assessment is usually not intended to be correct for 100% of the subjects to be diagnosed. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "subject" as used herein relates to animals, preferably mammals, and, more preferably, humans. The subject may be a male or female subject. Preferably, the subject is older than 40 years. The subject according to the present invention shall suffer from ischemic stroke. More preferably, said subject does not suffer from acute infections. Also preferably, the subject in the context with the aforementioned method shall have normal kidney function. Furthermore, the subject is, preferably, a subject presenting to an emergency unit. Moreover, the subject is, preferably, not pregnant. It is further envisaged that the subject does not suffer from brain tumor and/or from subarachnoid hemorrhage. This all applies to the subject to be tested and the reference subject.

As laid out herein below in more detail, the subject shall, preferably, not suffer from an acute cardiac event, in particular, an ACS. In particular, the subject does not suffer from an acute cardiac event at the onset of symptoms of stroke and did not suffer from an acute cardiac event before (preferably, within two days before) the onset of symptoms of stroke. Also, the subject, preferably does not suffer from an acute cardiac event at the time at which the sample is obtained.

The term "ischemic stroke" (herein also referred to as "stroke") is well understood by the skilled person (see e.g. Adams et al., Guidelines for the Early Management of Adults With Ischemic Stroke, A Guideline From the American Heart Association/ American Stroke Association Stroke Council, Clinical Cardiology Council, Cardiovascular Radiology and Intervention Council, and the Atherosclerotic Peripheral Vascular Disease and Quality of Care Outcomes in Research Interdisciplinary Working Groups in Stroke. 2007;38:1655 which is herewith incorporated by reference with respect to its entire disclosure content). As used herein, the term, preferably, refers to cerebral ischemic stroke. Moreover, it preferably refers to a stroke which is caused by reduced blood flow to the brain or parts thereof which leads to a reduced delivery (undersupply) of oxygen to brain cells. Preferably, the ischemic stroke is characterized by tissue anemia caused by obstruction of the inflow of arterial blood. Preferably, the ischemic stroke leads to irreversible tissue damage due to brain cell death. Ischemic stroke may be caused by atherothrombosis or embolism of a major cerebral artery, by coagulation disorders or non-atheromatous vascular disease, or by cardiac ischemia which leads to a reduced overall blood flow. The ischemic stroke is preferably selected from the group consisting of atherothrombotic stroke, cardioembolic stroke and lacunar stroke. Determination of the type stroke is known to the person skilled in the art and includes different imaging techniques such as echocardiography, electrocardiogram and doppler ultrasound. Preferably, the ischemic stroke is an acute ischemic stroke.

The term "ischemic stroke" does, preferably, not include hemorrhagic stroke.

In the context of the method of the present invention, the ischemic stroke may differ in its severity. For example, the stroke may be a major ischemic stroke or a minor stroke. Furthermore, the term "ischemic stroke" preferably also included transient ischemic attacks (TIAs). A TIA, preferably, is a brief episode of neurological dysfunction caused by insufficient cerebral blood supply to the brain that, preferably, lasts less than 2 hours, less than an hour, or less than 30 minutes without evidence of infarction.

The term "cardiac ischemia" as used herein, preferably, refers to a condition characterized by a reduced blood flow to the heart leading to a reduced delivery of oxygen delivery (undersupply) to cells comprised by the cardiac tissue. Preferably, cardiac tissue is affected by ischemia, if the amount of oxygen that is supplied to said tissue is not sufficient in order to cover the need of the cells comprised by said tissue, and, thus, to meet the rate of mitochondrial oxidation in said cells. Preferably, the cardiac ischemia is associated with or even caused by heart failure.

The term "heart failure" is well known in the art. As used herein, the term, preferably, relates to an impaired systolic and/or diastolic function of the heart being accompanied by overt signs of heart failure. Preferably, heart failure referred to herein is also chronic heart failure. Heart failure according to the present invention includes overt and/or advanced heart failure. In overt heart failure, the subject shows symptoms of heart failure as known to the person skilled in the art.

HF can be classified into various degrees of severity.

One classification is the so-called NYHA (New York Heart Association) classification. Heart failure patients are classified NYHA classes I, II, III and IV. A patient having heart failure has already experienced structural and functional changes to his pericardium, myocardium, coronary circulation or cardiac valves. He will not be able to fully restore his health, and is in need of a therapeutical treatment. Patients of NYHA Class I have no obvious symptoms of cardiovascular disease but already have objective evidence of functional impairment. Patients of NYHA class II have slight limitation of physical activity. Patients of NYHA class III show a marked limitation of physical activity. Patients of NYHA class IV are unable to carry out any physical activity without discomfort. They show symptoms of cardiac insufficiency at rest.

This functional classification is supplemented by the more recent classification by the American College of Cardiology and the American Heart Association (see J. Am. Coll. Cardiol. 2001;38;2101-2113, updated in 2005, see J. Am. Coll. Cardiol. 2005;46;e1-e82). 4 stages A, B, C and D are defined. Stages A and B are not HF but are considered to help identify patients early before developing "truly" HF. Stages A and B patients are best defined as those with risk factors for the development of HF. For example, patients with coronary artery disease, hypertension, or diabetes mellitus who do not yet demonstrate impaired left ventricular (LV) function, hypertrophy, or geometric chamber distortion would be considered stage A, whereas patients who are asymptomatic but demonstrate LV hypertrophy and/or impaired LV function would be designated as stage B. Stage C then denotes patients with current or past symptoms of HF associated with underlying structural heart disease (the bulk of patients with HF), and stage D designates patients with truly refractory HF.

As used herein, the term "heart failure", in particular, refers to stages C and D of the ACC/AHA classification referred to above. In these stages, the subject shows typical symptoms of heart failure. A subject having heart failure and being classified into stage C or D, preferably, has undergone permanent changes to his myocardium

The cardiac ischemia shall be chronic. "Chronic cardiac ischemia" as used herein refers to a persistent cardiac ischemia that has been present in the subject over a longer period of time. Accordingly, the term "chronic" as used herein, preferably, refers to cardiac ischemia that has been present in the subject for at least one month (in particular before the onset of symptoms of the stroke). Preferably, the cardiac ischemia has been present in the subject for at least three months (in particular before the onset of symptoms of the stroke). More preferably, the cardiac ischemia has been present in the subject for at least three months month (in particular before the onset of symptoms of the stroke). It is also envisaged that the cardiac ischemia has been present in the subject for one year or more (in particular before the onset of symptoms of the stroke). However, it is also contemplated that the chronic cardiac ischemia has been present in the subject for at least three days (in particular before the onset of symptoms of the stroke). This applies to the subject to be tested and the reference subject.

Since the cardiac ischemia in the context of the present invention is chronic, it is, particularly, envisaged that the cardiac ischemia is not acute. Accordingly, it is envisaged that the subject does not suffer from an acute cardiac event and/or does not and/or did not display symptoms of an acute cardiac event, in particular at the onset of symptoms of stroke and before the onset of symptoms of stroke. It is contemplated that the subject did not suffer from an acute cardiac event within two days before the onset of symptoms of stroke. In particular, the subject does not suffer from an acute cardiac event that is associated with or even caused the ischemic stroke.

This applies to the subject to be tested as well as to the reference subject(s), i.e. the subject(s) from which the reference amount is derived.

Thus, the term "chronic cardiac ischemia", preferably, does not encompass cardiac ischemia that is associated with or even caused by an acute cardiac event, in particular by an acute coronary syndrome (ACS). Accordingly, the subject suffering from ischemic stroke does not suffer from an acute coronary syndrome. ACS includes unstable angina pectoris (UAP) or myocardial infarction (MI). MI can be an ST-elevated MI (STEMI) or a non-ST-elevated MI (NSTEMI). Accordingly, in the context with present invention "cardiac ischemia" does not encompass cardiac ischemia associated with and/or caused by UAP, STEMI or NSTEMI.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

Preferably, the sample in the context with the aforementioned method shall have been obtained after the onset of symptoms of said ischemic stroke. In an embodiment, the sample has been obtained up to 72 hours after the onset of symptoms of ischemic stroke. In another embodiment the sample has been obtained up to 24 hours after the onset of ischemic stroke.

Preferably, the sample shall have been obtained not later than 12 or 24 hours after the onset of said symptoms. More preferably, the sample has been obtained not more than six or nine hours after the onset of symptoms of ischemic stroke. Even more preferably, the sample has been obtained not more than three hours after the onset of symptoms of ischemic stroke. It is particularly, envisaged that the sample is obtained at or after the first physical examination carried out after the onset of stroke symptoms such as when the subjects presents to an emergency physician or at the emergency room.

It is also contemplated that the sample has been obtained more than 24 hours, but not later than 72 hours after the onset of symptoms of ischemic stroke.

The term "soluble Flt-1" or "sFlt-1" (abbreviation for Soluble fms-like tyrosine kinase-1) as used herein, preferably, refers to polypeptide which is a soluble form of the VEGF receptor Flt1. It was identified in conditioned culture medium of human umbilical vein endothelial cells. The endogenous soluble Flt1 (sFlt-1) receptor is chromatographically and immunologically similar to recombinant human sFlt-1 and binds [125I] VEGF with a comparable high affinity. Human sFlt-1 is shown to form a VEGF-stabilized complex with the extracellular domain of KDR/Flk-1 in vitro. Preferably, sFlt-1 refers to human sFlt-1 as described in Kendall 1996, Biochem Biophs Res Commun 226(2): 324-328 (for amino acid sequences, see, e.g., also P17948, GI: 125361 for human and BAA24499.1, GI: 2809071 for mouse sFlt-1). The term also encompasses variants of the aforementioned human sFlt-1 polypeptides. Such variants have at least the same essential biological and immunological properties as the afore-mentioned sFlt-1 polypeptide. In particular, they share the same essential biological and immunological properties if they are detectable by the same specific assays referred to in this specification, e.g., by ELISA assays using polyclonal or monoclonal antibodies specifically recognizing the said sFlt-1 polypeptides. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific sFlt-1 polypeptide, preferably over the entire length of the human sFlt-1, respectively. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments or subunits of the specific sFlt-1 polypeptides or the aforementioned types of variants as long as these fragments have the essential immunological and biological properties as referred to above. Such fragments may be, e.g., degradation products of the sFlt-1 polypeptides. Further included are variants which differ due to posttranslational modifications such as phosphorylation or myristylation.

Determining the amount of a peptide or polypeptide referred to in this specification relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular re-sponses, ligands, labels, or enzymatic reaction products.

In accordance with the present invention, determining the amount of a peptide or polypeptide can be achieved by all known means for determining the amount of a peptide in a sample. Said means comprise immunoassay and methods which may utilize labeled molecules in various sandwich, competition, or other assay formats. Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on ElecsysTM analyzers), CBA (an enzymatic Cobalt Binding Assay, available for example on Roche-HitachiTM analyzers), and latex agglutination assays (available for example on Roche-HitachiTM analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) to the reference amount, it is possible to diagnose chronic cardiac ischemia in a subject suffering from ischemic stroke.

The term "reference amount" as used herein refers to an amount which allows for allocation of a subject into either the group of subjects suffering from chronic cardiac ischemia or into a group of subjects not suffering from chronic cardiac ischemia. Such a reference amount can be a threshold amount which separates these groups from each other. Preferably, the amount of the biomarker in sample of the test subject and the reference amount are determined with the same assay, e.g. with the assay described in the Examples section.

A suitable threshold amount separating the two groups can be calculated without further ado by the statistical tests referred to herein elsewhere based on amounts of the marker to be determined in the context of the present invention from either a subject or group of subjects suffering from ischemic stroke known to suffer from chronic cardiac ischemia or a subject or group of subjects suffering from ischemic stroke, but known not to suffer from chronic cardiac ischemia. Preferred referenced amounts which can be derived from the aforementioned subjects or group of subjects are indicated elsewhere herein.

In general, an amount of the marker(s) to be determined in the context of the present invention which is increased with respect to the reference amount, indicates that the subject to be tested suffers from chronic cardiac ischemia, whereas an amount of the marker to be determined in the context of the present invention which is decreased with respect to the reference amount, indicates that the subject to be tested does not suffer from chronic cardiac ischemia.

Preferably the reference amount is derived from a subject or a group of subjects suffering from an ischemic stroke, said subject(s) suffering from chronic cardiac ischemia, wherein an essentially identical amount or an increased amount of the marker(s), in particular of sFlt-1, in the test sample as compared to reference amount is indicative for chronic cardiac ischemia.

Also preferably, the reference amount is derived from a subject or a group of subjects suffering from an ischemic stroke, said subject(s) not suffering from chronic cardiac ischemia, wherein an essentially identical amount or an decreased amount of the marker(s), in particular of sFlt-1, in the test sample as compared to reference amount indicates that the subject does not suffer from chronic cardiac ischemia.

Preferably, reference amounts for sFlt-1 which are derived from a subject or a group of subjects suffering from an ischemic stroke, said subject(s) suffering from chronic cardiac ischemia, are within a range of 78 to 100 pg/ml, or, more preferably, within a range of 80 to 90 pg/ml. Preferably, the reference amount for sFlt-1 is 80 pg/ml, more preferably, the reference amount is 85 or 95 pg/ml.

Preferably, reference amounts for sFlt-1 which are derived from a subject or a group of subjects suffering from an ischemic stroke, said subject(s) not suffering from chronic cardiac ischemia, are within a range of 44 to 70 pg/ml, or, more preferably, within a range of 44 to 60 pg/ml. Preferably, the reference amount for sFlt-1 is 65 pg/ml, more preferably, the reference amount is 56 or 48 pg/ml.

Advantageously, it was shown in the context of the studies of the present invention that the determination of sFlt-1 in sample from a subject suffering from ischemic stroke allows for reliably diagnosing chronic cardiac ischemia in said subject. In general, the diagnosis of chronic cardiac ischemia is important, since it allows for making decisions with respect to further treatments. Once chronic cardiac ischemia has been diagnosed, therapies can be initiated that aim to increase cardiac output. By increasing cardiac output the perfusion, and, thus, the supply of the brain with oxygen can be increased, thereby reducing the risk of further brain damage, in particular infarction of the ischemic penumbra, that results from an undersupply with oxygen.

As set forth herein elsewhere, lowing the blood pressure is a frequent therapeutic measure in stroke patients in order to reduce the risk of secondary hemorrhage. However, lowering the bllod pressure will further reduce the supply of the brain with oxygen in patients suffering from chronic cardiac ischemia which may even lead to increased brain damage. Therefore, a therapy that lowers the blood pressure would out patients suffering from ischemic stroke and cardiac ischemia at further risk. Therefore, such a therapy should be avoided. Rather, treatment regimens should be initiated that aim to increase the cerebral perfusion, and, thus, the supply of the brain with oxygen.

In a preferred embodiment of the method of the present invention, the method further comprises the determination of a natriuretic peptide in a sample from the subject, and the comparison of the, thus, determined amount with a reference amount.

Accordingly, to the present invention relates to a method for diagnosing chronic cardiac ischemia in a subject suffering from ischemic stroke, said method comprising the steps of
a) determining the amounts of soluble fms-like tyrosine kinase-1 (sFlt-1) and of a natriuretic peptide in a sample from said subject, and
b) comparing the amounts of sFlt-1 and the natriuretic peptide to reference amounts, whereby chronic cardiac ischemia is diagnosed.

The further determination of a natriuretic peptide allows for increasing the specificity and sensitivity of the diagnosis of chronic cardiac ischemia.

Preferably, the amounts of the natriuretic peptide and sFlt-1 are determined in the same sample. However, it is also envisaged to determine the amounts of the markers in two different samples derived from the subject.

The term "natriuretic peptide" comprises Brain Natiuretic Peptide (BNP)-type peptides and variants thereof having the same predictive potential. Natriuretic peptides according to the present invention comprise BNP-type peptides and variants thereof (see e.g. Bonow, R. O. (1996). New insights into the cardiac natriuretic peptides. Circulation 93:1946-1950).

BNP-type peptides comprise pre-proBNP, proBNP, NT-proBNP, and BNP.

The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP).

Preferred natriuretic peptides according to the present invention are NT-proBNP, BNP, and variants thereof. BNP is the active hormone and has a shorter half-life than their respective inactive counterparts NT-proBNP, BNP is metabolized in the blood, whereas NT-proBNP circulates in the blood as an intact molecule and as such is eliminated renally. The in-vivo half-life of NT-proBNP is 120 min longer than that of BNP, which is 20 min (Smith M W, Espiner E A, Yandle T G, Charles C J, Richards A M. Delayed metabolism of human brain natriuretic peptide reflects resistance to neutral endopeptidase. J. Endocrinol. 2000; 167:239-46).

Preanalytics are more robust with NT-proBNP allowing easy transportation of the sample to a central laboratory (Mueller T, Gegenhuber A, Dieplinger B, Poelz W, Haltmayer M. Long-term stability of endogenous B-type natriuretic peptide (BNP) and amino terminal proBNP (NT-proBNP) in frozen plasma samples. Clin Chem Lab Med 2004; 42:942-4). Blood samples can be stored at room temperature for several days or may be mailed or shipped without recovery loss. In contrast, storage of BNP for 48 hours at room temperature or at 4°Celsius leads to a concentration loss of at least 20% (Mueller T, Gegenhuber A, et al., Clin Chem Lab Med 2004; 42:942-4, supra; Wu A H, Packer M, Smith A, Bijou R, Fink D, Mair J, Wallentin L, Johnston N, Feldcamp C S, Haverstick D M, Ahnadi C E, Grant A, Despres N, Bluestein B, Ghani F. Analytical and clinical evaluation of the Bayer ADVIA Centaur automated B-type natriuretic peptide assay in patients with heart failure: a multisite study. Clin Chem 2004; 50: 867-73). Therefore, depending on the time-course or properties of interest, either measurement of the active or the inactive forms of the natriuretic peptide can be advantageous.

The most preferred natriuretic peptides according to the present invention are NT-proBNP or variants thereof. As briefly discussed above, the human NT-proBNP as referred to in accordance with the present invention is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913, Bonow 1996, New Insights into the cardiac natriuretic peptides. Circulation 93: 1946-1950. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein.

The NT-proBNP referred to in accordance with the present invention further encompasses allelic and other variants of said specific sequence for human NT-proBNP discussed above. Specifically, envisaged are variant polypeptides which are on the amino acid level at least 60% identical, more preferably at least 70%, at least 80%, at least 90%, at least 95%, at least 98% or at least 99% identical, to human NT-proBNP, preferably, over the entire length. How to calculate the degree of identified between two sequence is described elsewhere herein. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be specifically recognizable (i.e. without cross reactions) by the aforementioned means or ligands used for determination of the amount of the natriuretic peptides. Biological and/or immunological NT-proBNP properties can be detected by the assay described in Karl et al. (Karl 1999, Development of a novel, N-Terminal-proBNP (NT-proBNP) assay with a low detection limit. Scand J Clin Invest 230:177-181), Yeo et al. (Yeo 2003, Multicenter evaluation of the Roche NT-proBNP assay and comparison to the Biosite Triage assay. Clinica Chimica Acta 338:107-115), and in the Example, below. Variants also include posttranslationally modified natriuretic peptides such as glycosylated peptides.

With respect to the reference amount for the natriuretic peptide and the diagnostic algorithm, the same applies as set forth with respect to the reference amount for sFlt-1. Thus, the reference amount for the natriuretic peptide may be derived from the same subject or group of subjects (to be more precise from a sample/samples from the same subject or group of subjects) as the reference amount for sFlt-1.

Preferably the reference amounts for sFlt-1 and the natriuretic peptide, in particular of Nt-proBNP, are derived from a subject or a group of subjects suffering from an ischemic stroke, said subject(s) suffering from chronic cardiac ischemia, wherein an essentially identical amount or an increased amount of the markers in the test sample as compared to reference amounts is indicative for chronic cardiac ischemia.

Also preferably, the reference amounts for sFlt-1 and the natriuretic peptide, in particular of NT-proBNP, are derived from a subject or a group of subjects suffering from an ischemic stroke, said subject(s) not suffering from chronic cardiac ischemia, wherein an essentially identical amount or an decreased amount of the markers in the test sample as compared to reference amounts indicates that the subject does not suffer from chronic cardiac ischemia.

Preferably, reference amounts for NT-proBNP which are derived from a subj ect or a group of subjects suffering from an ischemic stroke, said subject(s) suffering from chronic cardiac ischemia, are within a range of 287 to 2171 pg/ml, or, more preferably, within a range of 500 to 700 pg/ml. Preferably, the reference amount for NT-proBNP is 700 pg/ml, more preferably, the reference amount is 600 or 531 pg/ml.

Preferably, reference amounts for NT-proBNP which are derived from a subj ect or a group of subjects suffering from an ischemic stroke, said subject(s) not suffering from chronic cardiac ischemia, are within a range of 50 to 250 pg/ml, or, more preferably, within a range of 100 to 200 pg/ml. Preferably, the reference amount for NT-proBNP is 200 pg/ml, more preferably, the reference amount is 100 or 83 pg/ml.

In an aspect of the invention, a method for establishing an aid for diagnosing whether a subject who suffers from ischemic stroke suffers from chronic cardiac ischemia, or not, is contemplated, said method comprising:
a) determining the amount of a sFlt-1 in a sample of said subject, said determining comprises (i) bringing the sample into contact with a detection agent that specifically binds to the sFlt-1 for a time sufficient to allow for the formation of a complex of the said detection agent and the sFlt-1 from the sample, (ii) measuring the amount of the formed complex, wherein the said amount of the formed complex is proportional to the amount of the sFlt-1 present in the sample, and (iii) transforming the amount of the formed complex into an amount of the sFlt-1 reflecting the amount of the sFlt-1 present in the sample;
b) comparing said amount to a reference amount; and
c) establishing an aid for diagnosing aid for diagnosing whether the subject suffers from chronic cardiac ischemia based on the result of the comparison made in step b).

A suitable detection agent may be, in an aspect, an antibody which is specifically binds to a sFlt-1, in a sample of a subject to be investigated by the method of the invention. Another detection agent that can be applied, in an aspect, may be an aptamere which specifically binds to the sFlt-1 in the sample. In yet an aspect the, sample is removed from the complex formed between the detection agent and the sFlt-1 prior to the measurement of the amount of formed complex. Accordingly, in an aspect, the detection agent may be immobilized on a solid support. In yet an aspect, the sample can be removed from the formed complex on the solid support by applying a washing solution. The formed complex shall be proportional to the amount of the sFlt-1 present in the sample. It will be understood that the specificity and/or sensitivity of the detection agent to be applied defines the degree of proportion of sFlt-1 comprised in the sample which is capable of being specifically bound. Further details on how the determination can be carried out are also found elsewhere herein. The amount of formed complex shall be transformed into an amount of sFlt-1 reflecting the amount indeed present in the sample. Such an amount, in an aspect, may be essentially the amount present in the sample or may be, in another aspect, an amount which is a certain proportion thereof due to the relationship between the formed complex and the amount present in the original sample.

Optionally, a natriuretic peptide may be determined in the context of the aforementioned methods. The natriuretic peptide may be determined as described for sFlt-1.

In yet an aspect of the aforementioned method, step a) may be carried out by an analyzing unit, in an aspect, an analyzing unit as defined elsewhere herein.

In an aspect of the method of the invention, the amount determined in step a) is compared to a reference amount. In an aspect, the reference is a reference as defined elsewhere herein. In yet another aspect, the reference takes into account the proportional relationship between the measured amount of complex and the amount present in the original sample. Thus, the references applied in an aspect of the method of the invention are artificial references which are adopted to reflect the limitations of the detection agent that has been used. In another aspect, said relationship can be also taken into account when carrying out the comparison, e.g., by including a normalization and/or correction calculation step for the determined amount prior to actually comparing the value of the determined amount and the reference. Again, the normalization and/or correction calculation step for the determined amount adopts the comparison step such that the limitations of the detection agent that has been used are reflected properly. In an aspect, the comparison is carried out automatically, e.g., assisted by a computer system or the like.

The aid for diagnosing is established based on the comparison carried out in step b) by allocating the subject either into a group of subjects suffering from chronic cardiac ischemia with certain likelihood or a group of subjects not suffering therefrom. As discussed elsewhere herein already, the allocation of the investigated subject must not be correct in 100% of the investigated cases. Moreover, the groups of subjects into which the investigated subject is allocated are artificial groups in that they are established based on statistical considerations, i.e. a certain preselected degree of likelihood based on which the method of the invention shall operate. Thus, the method may establish an aid of diagnosis which may, in an aspect, require further strengthening of the diagnosis by other techniques. In an aspect of the invention, the aid for diagnosing is established automatically, e.g., assisted by a computer system or the like.

In an aspect of the method of the invention, said method further comprises a step of recommending and/or managing the subject according to the result of the aid of diagnosis established in step c). Preferably, a therapy is recommended as set forth elsewhere herein in detail. In particular, a subject who does not suffer from chronic cardiac ischemia is susceptible to a therapy that lowers to blood pressure. Moreover, a subject who suffers from chronic cardiac ischemia is not susceptible to a therapy that lowers to blood pressure. However, said subject is susceptible to a therapy that increases cerebral perfusion.

In an aspect of the aforementioned method, steps b) and/or c) are carried out by an evaluation unit as set forth elsewhere herein.

### Method for predicting in a subject suffering from ischemic stroke the risk to suffer from infarction of the ischemic penumbra

The definitions and explanations given herein above apply *mutatis mutandis* to the following (except if stated otherwise).

The present invention also relates to method for predicting in a subject suffering from ischemic stroke the risk to suffer from infarction of the ischemic penumbra, the method comprising
a) determining the amount of soluble fins-like tyrosine kinase-1 (sFlt-1) in a sample from said subject, and
b) comparing the amount of sFlt-1 in said sample to a reference amount, whereby the risk to suffer from infarction of the ischemic penumbra is predicted.

In a preferred embodiment of the method of the present invention, the method further comprises the determination of a natriuretic peptide in a sample from the subject, and, the comparison of the, thus, determined amount with a reference amount for the natriuretic peptide.

The term "ischemic penumbra" is well understood by the skilled person. Within the ischemic cerebrovascular bed, there are two major zones of injury: the core ischemic zone and the "ischemic penumbra". After the ischemic stroke, blood flow and therefore oxygen supply is reduced locally, leading to hypoxia of the cells near the location of the original insult. This can lead to cell death and amplify the original damage from the infarction. However, the penumbra area may remain viable for several hours after an ischemic event due to the collateral arteries that supply the penumbral zone. It is understood by the skilled person that subjects suffering from cardiac ischemia are at increased risk of suffering from infarction of the ischemic penumbra.

In the context of the present invention, the "ischemic penumbra" is ischemic brain tissue surrounding the core region of the infarction. This brain tissue is dysfunctional, but still viable. In contrast, the brain tissue of the core region is not viable anymore. If sufficient supply with oxygen is restored, the ischemic penumbra becomes functional again. Accordingly, the ischemic penumbra is reversibly dysfunctional. The penumbra region typically occurs when blood flow drops below 20 mL/100 g/min.

If sufficient supply of the ischemic penumbra with oxygen is not restored, it becomes infarcted. Thus, the brain tissue affected by ischemic penumbra dies. Accordingly, the term "infarction", preferably, refers to tissue death of brain tissue resulting from lack of oxygen supply. Whether the tissue has become infarcted or not can be assessed by the skilled person without further ado, e.g., by MRT.

The term "predicting the risk" as used herein, preferably, refers to assessing the probability according to which a subject who suffers from ischemic stroke will suffer from infarction of the ischemic penumbra. More preferably, the risk/probability of infarction of the ischemic penumbra within a certain time window is predicted. In a preferred embodiment of the present invention, the predictive window, preferably, is an interval of at least 30 minutes, of at least 60 minutes, of at least 2 hours, of at least 3 hours, or at least 6 hours or any intermitting time range. In a particular preferred embodiment of the present invention, the predictive window, preferably, is a predictive window of 1 hour to 10 hours, or more preferably, of 1 hour to 6 hours, and most preferably, of 30 minutes to 6 hours. Preferably, said predictive window is calculated from the time point at which the sample to be tested has been obtained.

As will be understood by those skilled in the art, such a prediction is usually not intended to be correct for 100% of the subjects. The term, however, requires that prediction can be made for a statistically significant portion of subjects in a proper and correct manner. Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98%, or at least 99%. The p-values are, preferably, 0.1, 0.05, 0.01, 0.005, or 0.0001. Preferably, the probability envisaged by the present invention allows that the prediction of an increased, normal or decreased risk will be correct for at least 60%, at least 70%, at least 80%, or at least 90% of the subjects of a given cohort or population. The term, preferably, relates to predicting whether a subject is at elevated risk or reduced risk as compared to the average risk for the infarction of the ischemic penumbra in a population of subjects.

The term "predicting the risk of to suffer from infarction of the ischemic penumbra" as used herein means that the subject to be analyzed by the method of the present invention is allocated either into the group of subjects being at risk of infarction of the ischemic penumbra, or into the group of subjects being not at risk of infarction of the ischemic penumbra. A risk of infarction of the ischemic penumbra as referred to in accordance with the present invention, preferably, means that the risk of infarction of the ischemic penumbra is elevated (within the predictive window). Preferably, said risk is elevated as compared to the average risk in a cohort of subjects with ischemic stroke. If a subject is not at risk of infarction of the ischemic penumbra as referred to in accordance with the present invention, preferably, the risk of infarction of the ischemic penumbra shall be reduced (within the predictive window). Preferably, said risk is reduced as compared to the average risk in a cohort of subjects with ischemic stroke. A subject who is at risk of infarction of the ischemic penumbra preferably has a risk of 60% or larger, or, more preferably of 75% or larger of infarction of the ischemic penumbra, preferably, within the predictive window. A subj ect who is at not at risk of infarction of the ischemic penumbra preferably has a risk of lower than 30%, more preferably of lower than, 20 % or lower of infarction of the ischemic penumbra preferably, within the predictive window.

The term "reference amount" as used in the context with the aforementioned method refers to amounts of the biomarker sFlt-1 (and optionally a natriuretic peptide) which allow for predicting whether a subject is at risk of infarction of the ischemic penumbra, or not. Therefore, the reference may either be derived from (i) a subject suffering from ischemic stroke known to be at risk of infarction of the ischemic penumbra (or from a group of said subjects) or (ii) a subject suffering from ischemic stroke known not to be at risk of infarction of the ischemic penumbra. Preferably, said reference is derived from a sample of the aforementioned subjects. More preferably, an increased amount of the biomarker sFlt-1 (and optionally a natriuretic peptide) compared to the reference amount is indicative for a subject being at risk of infarction of the ischemic penumbra, whereas a decreased amount of the biomarker sFlt-1 (and optionally a natriuretic peptide) compared to the reference amount is indicative for a subject not being at risk of infarction of the ischemic penumbra.

A preferred diagnostic algorithm is as follows:
Preferably the reference amount is derived from a subject or a group of subjects suffering from an ischemic stroke, said subject(s) being at risk to suffer from infarction of the ischemic penumbra, wherein an essentially identical amount or an increased amount of the marker(s), in particular of sFlt-1, in the test sample as compared to reference amount is indicative for a subject who is at risk to suffer from infarction of the ischemic penumbra.
Also preferably, the reference amount is derived from a subject or a group of subjects suffering from an ischemic stroke, said subject(s) not being at risk to suffer from infarction of the ischemic penumbra, wherein an essentially identical amount or an decreased amount of the marker(s), in particular of sFlt-1, in the test sample as compared to reference amount indicates that the subject is not at risk to suffer from infarction of the ischemic penumbra.

If both the amounts of sFlt-1 and the natriuretic peptide are determined, the following applies:
Preferably the reference amounts for sFlt-1 and the natriuretic peptide are derived from a subject or a group of subjects suffering from an ischemic stroke, said subject(s) being at risk to suffer from infarction of the ischemic penumbra, wherein an essentially identical amount or an increased amount of the markers in the test sample as compared to reference amounts is indicative for a subject who is at risk to suffer from infarction of the ischemic penumbra.
Also preferably, the reference amounts for sFlt-1 and the natriuretic peptide are derived from a subject or a group of subjects suffering from an ischemic stroke, said subj ect(s) not being at risk to suffer from infarction of the ischemic penumbra, wherein an essentially identical amount or an decreased amount of the markers in the test sample as compared to reference amounts indicates that the subject is not at risk to suffer from infarction of the ischemic penumbra.
Preferably, reference amounts for sFlt-1 which are derived from a subject or a group of subjects suffering from an ischemic stroke, said subject(s) being at risk to suffer from infarction of the ischemic penumbra, are within a range of 78 to 100 pg/ml, or, more preferably, within a range of 80 to 90 pg/ml. Preferably, the reference amount for sFlt-1 is 80 pg/ml, more preferably, the reference amount is 85 or 95 pg/ml.
Preferably, reference amounts for sFlt-1 which are derived from a subject or a group of subjects suffering from an ischemic stroke, said subject(s) being not at risk to suffer from infarction of the ischemic penumbra, are within a range of 44 to 70 pg/ml, or, more preferably, within a range of 44 to 60 pg/ml. Preferably, the reference amount for sFlt-1 is 65 pg/ml, more preferably, the reference amount is 56 or 48 pg/ml.
Preferably, reference amounts for NT-proBNP which are derived from a subj ect or a group of subjects suffering from an ischemic stroke, said subject(s) being at risk to suffer from infarction of the ischemic penumbra, are within a range of 287 to 2171 pg/ml, or, more preferably, within a range of 500 to 700 pg/ml. Preferably, the reference amount for NT-proBNP is 700 pg/ml, more preferably, the reference amount is 600 or 531 pg/ml.
Preferably, reference amounts for NT-proBNP which are derived from a subj ect or a group of subjects suffering from an ischemic stroke, said subject(s) not being at risk to suffer from infarction of the ischemic penumbra, are within a range of 50 to 250 pg/ml, or, more preferably, within a range of 100 to 200 pg/ml. Preferably, the reference amount for NT-proBNP is 200 pg/ml, more preferably, the reference amount is 100 or 83 pg/ml.

### Method for deciding whether a subject who suffers from ischemic stroke is susceptible to a therapy that lowers to blood pressure and/or to a therapy that increases cerebral perfusion

The definitions and explanations given herein above apply *mutatis mutandis* to the following (except if stated otherwise).

Moreover, the present invention relates to a method for deciding whether a subject who suffers from ischemic stroke is susceptible to a therapy that lowers to blood pressure and/or to a therapy that increases cerebral perfusion, the method comprising the steps of
a) determining the amount of soluble fms-like tyrosine kinase-1 (sFlt-1) in a sample from said subject, and
b) comparing the amount of sFlt-1 in said sample to a reference amount, whereby is it decided whether the subject is susceptible to a therapy that lowers to blood pressure and/or to a therapy that increases cerebral perfusion.

In a preferred embodiment of the method of the present invention, the method further the determination of a natriuretic peptide in a sample from the subject, and, the comparison of the, thus, determined amount with a reference amount for the natriuretic peptide.

Preferably, a subject being susceptible to a therapy as set forth in the context of the afore-mentioned method, i.e. a therapy for increasing cerebral perfusion and/or to a therapy that lowers to blood pressure, is identified based on the result of the comparison carried out in step b).

The phrase "deciding whether a subject being susceptible to a therapy" as used herein means assessing whether a subject suffering from an ischemic stroke will be eligible to said therapy or not. Accordingly, by deciding whether a subject being susceptible to a therapy, a subject can be identified who is susceptible to a therapy as set forth in the context of the aforementioned method, or not.

A subject who is susceptible to a therapy for lowering the blood pressure is, preferably, not at risk of suffering from adverse side effects of said therapy if said therapy is initiated or continued, whereas a subject who is not susceptible to said therapy will be at risk of suffering from adverse side effects of the said therapy if said therapy is initiated or continued. Adverse side effects of a therapy for lowering the blood pressure are, preferably, further necrosis of brain tissue. In particular, an adverse side effect is the infarction of the ischemic penumbra as described herein elsewhere.

A subject who is susceptible to a therapy for increasing cerebral perfusion will, preferably, benefit from said therapy if said therapy initiated or continued. Increasing cerebral perfusion with allow for increasing the supply of the brain with oxygen and, thus, may prevent further adverse side effects such as infarction of the ischemic penumbra.

As will be understood by those skilled in the art, the assessment whether a subject is susceptible to said therapies is usually not intended to be correct for all (i.e. 100%) of the subjects to be identified. The term, however, requires that a statistically significant portion of subjects can be identified (e.g. a cohort in a cohort study). Whether a portion is statistically significant can be determined without further ado by the person skilled in the art using various well known statistic evaluation tools, e.g., determination of confidence intervals, p-value determination, Student's t-test, Mann- Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. More preferably, at least 60%, at least 70%, at least 80% or at least 90% of the subjects of a population can be properly identified by the method of the present invention.

The therapy for lowering the blood pressure may have been initiated before or after the onset of stroke symptoms.

In case that the therapy for lowering the blood pressure has been initiated after the onset of symptoms of ischemic stroke (e.g. shortly after presenting at the emergency room), said therapy, preferably, shall have been initiated within one to 48 hours, within one to 24 hours, within one to twelve hours, or within one to six hours, or, more preferably, within one to three hours after the onset of symptoms of ischemic stroke.

Therapies for lowering the blood pressure in patients with ischemic stroke are well known in the art. Particularly preferred therapies lowering the blood pressure are, e.g. disclosed by Adams et al. (loc. cit.). Preferably, the therapy for lowering the blood pressure shall lower arterial blood pressure. Also preferably, the therapy for lowering the blood pressure is selected from the group consisting of administration of a blood pressure lowering drug. Blood pressure lowering drugs are well known in the art. Preferred blood pressure lowering drugs are selected from the group consisting of administration of ACE inhibitors, administration of beta adrenergic blockers, administration of angiotensin-II receptor antagonists, administration of diuretics.

ACE-inhibitors are known to the person skilled in the art. Examples include benazepril, captopril, cilazapril, enalapril, fosinopril, lisinopril, moexipril, perindopril, quinapril, ramipril, spirapril, and trandolapril.

Beta adrenergic blockers (non-selective and βi -selective) are known to the person skilled in the art. Examples include acebutolol, alprenolol, atenolol, betaxolol, bisoprolol, bupranolol, carazolol, carteolol, carvedilol, celiprolol, metipranolol, metoprolol, nadolol, nebivolol, oxprenolol, penbutolol, pindolol, propanolol, sotalol, tanilolol, and timolol.

Preferred angiotensin-II receptor antagonists (also known as angiotensin receptor blockers (ARBs), AT1-receptor antagonists or sartan), are Valsartan, Telmisartan, Losartan, Irbesartan, Irbesartan, Azilsartan, and Olmesartan.

Preferred diuretics are loop diuretics. Also preferred are thiazides.

Therapies for increasing cerebral perfusion are well known in the art. Preferably, the therapy is selected from a therapy that increases blood pressure, intravenous thrombolysis, in particular within three hours after the onset of symptoms of stroke, intra- and extracranial endovascular intervention (preferably by stenting, more preferably stening of arteria carotis interna, antithrombotic treatment and anticoagulation (preferably, by heparin and Vitamin K antagonists). If case if no thrombolysis or after thrombolysis antithrombotic therapy can be initiated, using a variety of drugs including aspirin, heparin, vitamin K antagonists or newer drugs that inhibit Thrombin (dabigatran of factor Xa (rivaroxaban and apixaban). The choice of drugs may depends on the stroke risk and concomitant disorders as well as bleeding risk (for detaidl see Skanes A.V. et al Canadian Journal of Cardiology 2012: 28: 125 - 136).

Preferably, the therapy for increasing blood pressure is applied in a subject with low blood pressure, in particular in a subject having a systolic blood pressure of preferably lower than 120 or 110 mmHg, or more preferably, of lower than 100 or 90 mmHg, and, most preferably, of lower than 80 mmHg. Therapies for increasing blood pressure are well known in the art. Preferably, the therapy for increasing blood pressure is selected from the group consisting of administration of a blood pressure increasing drug, in particular of phenylephrine, midodrine, fludrocortisone, NaCl, dopamine, diaspirin, cross-linked hemoglobin, or low-molecular dextrans.

Also encompassed by the present invention is that the therapy for increasing the blood pressure is a discontinuation of administration of blood pressure lowering drugs. This applies, if the subject is already treated with blood pressure lowering drugs when suffering from stroke. Preferred blood pressure lowering drugs are disclosed herein above.

The term "reference amount" as used in the context with the aforementioned method refers to amounts of the biomarker sFlt-1 (and optionally a natriuretic peptide) which allow for deciding whether a subject is susceptible to a therapy as set forth in connection with the aforementioned method. Therefore, the reference may either be derived from (i) a subject suffering from ischemic stroke known to susceptible to a therapy as set forth in connection with the aforementioned method (or from a group of said subjects) or (ii) a subject suffering from ischemic stroke known not to be susceptible to a therapy as set forth in connection with the aforementioned method. Preferably, said reference is derived from a sample of the aforementioned subjects.

If the therapy is a therapy for increasing cerebral perfusion, the following applies in case of the determination of sFlt-1:
Preferably, i) the reference amount is derived from a subject or a group of subjects suffering from an ischemic stroke, wherein said subject(s) are known to be susceptible for a therapy for increasing cerebral perfusion, wherein an essentially identical amount or an increased amount of sFlt-1 in the test sample as compared to reference amount is indicative for a subject who is susceptible to a therapy for increasing cerebral perfusion and/or ii) the reference amount is derived from a subject or a group of subjects suffering from an ischemic stroke, wherein said subject(s) are known not to be susceptible to a therapy for increasing cerebral perfusion, wherein an essentially identical amount or a decreased amount of sFlt-1 in the test sample as compared to reference amount is indicative for a subject who is not susceptible to a therapy for increasing cerebral perfusion.

If the therapy is a therapy for increasing cerebral perfusion, the following applies in case of the combined determination of sFlt-1 and a natriuretic peptide:
Preferably, i) the reference amounts for sFlt-1 and the natriuretic peptide are derived from a subject or a group of subjects suffering from an ischemic stroke, wherein said subject(s) are known to be susceptible for a therapy for increasing cerebral perfusion, wherein an essentially identical amount or an increased amount of sFlt-1 and the natriuretic peptide in the test sample as compared to reference amount is indicative for a subject who is susceptible to a therapy for increasing cerebral perfusion and/or ii) the reference amounts for sFlt-1 and the natriuretic peptide are derived from a subject or a group of subjects suffering from an ischemic stroke, wherein said subject(s) are known not to be susceptible to a therapy for increasing cerebral perfusion, wherein an essentially identical amount or a decreased amount of sFlt-1 and the natriuretic peptide in the test sample as compared to reference amount is indicative for a subject who is not susceptible to a therapy for increasing cerebral perfusion.

Preferably, reference amounts for sFlt-1 which are derived from a subject or a group of subjects suffering from an ischemic stroke, said subject(s) are known to be susceptible for a therapy for increasing cerebral perfusion, are within a range of 78 to 100 pg/ml, or, more preferably, within a range of 80 to 90 pg/ml. Preferably, the reference amount for sFlt-1 is 80 pg/ml, more preferably, the reference amount is 85 or 95 pg/ml.

Preferably, reference amounts for sFlt-1 which are derived from a subject or a group of subjects suffering from an ischemic stroke, said subject(s) are known not to be susceptible to a therapy for increasing cerebral perfusion, are within a range of 44 to 70 pg/ml, or, more preferably, within a range of 44 to 60 pg/ml. Preferably, the reference amount for sFlt-1 is 65 pg/ml, more preferably, the reference amount is 56 or 48 pg/ml.

Preferably, reference amounts for NT-proBNP which are derived from a subj ect or a group of subjects suffering from an ischemic stroke, said subject(s) are known to be susceptible to a therapy for increasing cerebral perfusion, are within a range of 287 to 2171 pg/ml, or, more preferably, within a range of 500 to 700 pg/ml. Preferably, the reference amount for NT-proBNP is 700 pg/ml, more preferably, the reference amount is 600 or 531 pg/ml. Preferably, reference amounts for NT-proBNP which are derived from a subj ect or a group of subjects suffering from an ischemic stroke, said subject(s) are known not to be susceptible to a therapy for increasing cerebral perfusion, are within a range of 50 to 250 pg/ml, or, more preferably, within a range of 100 to 200 pg/ml. Preferably, the reference amount for NT-proBNP is 200 pg/ml, more preferably, the reference amount is 100 or 83 pg/ml.

If the therapy is a therapy that lowers the blood pressure, the following applies in case of the determination of sFlt-1:
Preferably, i) the reference amount is derived from a subject or a group of subjects suffering from an ischemic stroke, wherein said subject(s) are known to be susceptible for a therapy for lowering the blood pressure, wherein an essentially identical amount or a decreased amount of sFlt-1 in the test sample as compared to reference amount is indicative for a subject who is susceptible to a therapy that lowers the blood pressure and/or wherein ii) the reference amount is derived from a subject or a group of subjects suffering from an ischemic stroke, wherein said subject(s) are known not to be susceptible to a therapy that lowers the blood pressure, wherein an essentially identical amount or an increased amount of sFlt-1 in the test sample as compared to reference amount is indicative for a subject who is not susceptible to a therapy that lowers the blood pressure.

If the therapy is a therapy that lowers the blood pressure, the following applies in case of the combined determination of sFlt-1 and a natriuretic peptide.

Preferably, i) the reference amounts for sFlt-1 and the natriuretic peptide are derived from a subject or a group of subjects suffering from an ischemic stroke, wherein said subject(s) are known to be susceptible for a therapy for lowering the blood pressure, wherein an essentially identical amount and/or a decreased amount of sFlt-1 and the natriuretic peptide in the test sample as compared to reference amount is indicative for a subject who is susceptible to a therapy that lowers the blood pressure and/or wherein ii) the reference amounts for sFlt-1 and the natriuretic peptide are derived from a subject or a group of subjects suffering from an ischemic stroke, wherein said subject(s) are known not to be susceptible to a therapy that lowers the blood pressure, wherein an essentially identical amount or an increased amount of the markers in the test sample as compared to reference amount is indicative for a subject who is not susceptible to a therapy that lowers the blood pressure.

Preferably, reference amounts for sFlt-1 which are derived from a subject or a group of subjects suffering from an ischemic stroke, said subject(s) are known not to be susceptible for a therapy for lowering the blood pressure, are within a range of 78 to 100 pg/ml, or, more preferably, within a range of 80 to 90 pg/ml. Preferably, the reference amount for sFlt-1 is 80 pg/ml, more preferably, the reference amount is 85 or 95 pg/ml.

Preferably, reference amounts for sFlt-1 which are derived from a subject or a group of subjects suffering from an ischemic stroke, said subject(s) are known to be susceptible for a therapy for lowering the blood pressure, are within a range of 44 to 70 pg/ml, or, more preferably, within a range of 44 to 60 pg/ml. Preferably, the reference amount for sFlt-1 is 65 pg/ml, more preferably, the reference amount is 56 or 48 pg/ml.

Preferably, reference amounts for NT-proBNP which are derived from a subj ect or a group of subjects suffering from an ischemic stroke, said subject(s) are known not to be susceptible for a therapy for lowering the blood pressure, are within a range of 287 to 2171 pg/ml, or, more preferably, within a range of 500 to 700 pg/ml. Preferably, the reference amount for NT-proBNP is 700 pg/ml, more preferably, the reference amount is 600 or 531 pg/ml.

Preferably, reference amounts for NT-proBNP which are derived from a subject or a group of subjects suffering from an ischemic stroke, said subject(s) are known to be susceptible for a therapy for lowering the blood pressure, are within a range of 50 to 250 pg/ml, or, more preferably, within a range of 100 to 200 pg/ml. Preferably, the reference amount for NT-proBNP is 200 pg/ml, more preferably, the reference amount is 100 or 83 pg/ml.

Further, the present invention relates to the use of the biomarker sFlt1, or of a detection agent which specifically binds thereto in a sample of a subject suffering from an ischemic stroke for diagnosing chronic cardiac ischemia in said subject.

Further, the present invention relates to the use of i) the biomarker sFlt1 and of a natriuretic petide, or ii) a detection agent which specifically binds to sFlt-1 and of a detection agent which specifically binds to a natriuretic peptide, in a sample of a subject suffering from an ischemic stroke for diagnosing chronic cardiac ischemia in said subject.

The present invention also relates to the use of i) the biomarker sFlt1 and of a natriuretic petide, or ii) a detection agent which specifically binds to sFlt-1 and of a detection agent which specifically binds to a natriuretic peptide, in a sample of a subject suffering from ischemic stroke for predicting the risk of the subject to suffer from infarction of the ischemic penumbra.

Further, the present invention relates to the use of the the biomarker sFlt1, or of a detection agent which specifically binds thereto, in a sample of a subject suffering from an ischemic stroke for deciding whether the subject is susceptible to a therapy that lowers to blood pressure and/or to a therapy that increases cerebral perfusion.

Further, the present invention relates to the use of i) the biomarker sFlt1 and of a natriuretic petide, or ii) a detection agent which specifically binds to sFlt-1 and a detection agent which specifically binds to a natriuretic peptide, in a sample of a subject suffering from an ischemic stroke for deciding whether the subject is susceptible to a therapy that lowers to blood pressure and/or to a therapy that increases cerebral perfusion.

The term "detection agent" as used herein refers to an agent which is capable of specifically recognizing and binding to one of the biomarkers referred to above when present in a sample, i.e. to sFlt1 or to a natriuretic peptide. Moreover, the said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamers which specifically binds to the biomarker. The term "antibody" has been described elsewhere herein.

The present invention also pertains to a device adapted for carrying out the method of the present invention for diagnosing chronic cardiac ischemia in a subject suffering from ischemic stroke, comprising
a) an analyzing unit comprising a detection agent which specifically binds to sFlt1 (and optionally of a natriuretic peptide), said unit being adapted for determining the amount of sFlt-1 (and optionally of a natriuretic peptide) in a sample of a said subject; and
b) an evaluation unit for comparing the determined amount(s) with a reference amount(s) whereby chronic cardiac ischemia is diagnosed, said unit comprising a database with reference amount values (a reference amount value) derived from a subject (or group of subjects) as defined in the context of the method for diagnosing chronic cardiac ischemia and a computer-implemented algorithm for carrying out a comparison as specified in the context of said method.

The present invention also pertains to a device adapted for carrying out the method of the present invention for predicting the risk to suffer from infarction of the ischemic penumbra in a subject suffering from ischemic stroke, comprising
a) an analyzing unit comprising a detection agent which specifically binds to sFlt1 (and optionally of a natriuretic peptide), said unit being adapted for determining the amount of sFlt-1 (and optionally of a natriuretic peptide) in a sample of a said subject; and
b) an evaluation unit for comparing the determined amount(s) with a reference amount(s) whereby the risk is predicted, said unit comprising a database with reference amount values (a reference amount value) derived from a subj ect (or group of subjects) as defined in the context of the said method, and a computer-implemented algorithm for carrying out a comparison as specified in the context of said method.

The present invention also pertains to a device adapted for carrying out the method of the present invention for deciding whether a subject who suffers from ischemic stroke is susceptible to a therapy that lowers to blood pressure and/or to a therapy that increases cerebral perfusion, comprising
a) an analyzing unit comprising a detection agent which specifically binds to sFlt1 (and optionally of a natriuretic peptide), said unit being adapted for determining the amount of sFlt-1 (and optionally of a natriuretic peptide) in a sample of a said subject; and
b) an evaluation unit for comparing the determined amount(s) with a reference amount(s) whereby it can be decided whether the subject is susceptible to a therapy that lowers to blood pressure and/or to a therapy that increases cerebral perfusion, said unit comprising a database with reference amount values (a reference amount value) derived from a subject (or group of subjects) as defined in the context of the said method, and a computer-implemented algorithm for carrying out a comparison as specified in the context of said method.

The term "device" as used herein relates to a system comprising the aforementioned units operatively linked to each other as to allow the diagnosis or monitoring according to the methods of the invention. Preferred detection agents which can be used for the analysing unit are disclosed elsewhere herein. The analysing unit, preferably, comprises said detection agents in immobilized form on a solid support which is to be contacted to the sample comprising the biomarkers the amount of which is to be determined. Moreover, the analysing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references are either derived from a subject or group of subjects as defined above in context with the method of the present invention. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician.

It follows from the above that according to some embodiments of the instant disclosure, portions of some steps of methods disclosed and described herein may be performed by a computing device. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants ("PDA"), such as BLACKBERRY brand devices, cellular devices, tablet computers, servers, and the like. In general, a computing device comprises a processor capable of executing a plurality of instructions (such as a program of software).

A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device.

According to embodiments of the instant disclosure, software may include instructions which, when executed by a processor of the computing device, may perform one or more steps of the methods disclosed herein. Some of the instructions may be adapted to produce signals that control operation of other machines and thus may operate through those control signals to transform materials far removed from the computer itself. These descriptions and representations are the means used by those skilled in the art of data processing, for example, to most effectively convey the substance of their work to others skilled in the art. The plurality of instructions may also comprise an algorithm which is generally conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

The present invention contemplates a kit adapted for carrying out the method of the present invention for diagnosing chronic cardiac ischemia associated with ischemic stroke in a subject suffering from ischemic stroke, said kit comprising a detection agent for the biomarker sFlt-1 as well as instructions for carrying out the said method.

The present invention contemplates a kit adapted for carrying out the method of the present invention for deciding whether a subj ect who suffers from ischemic stroke is susceptible to a therapy that lowers to blood pressure and/or to a therapy that increases cerebral perfusion, said kit comprising a detection agent for the biomarker sFlt-1 as well as instructions for carrying out the said method.

The present invention contemplates a kit adapted for carrying out the method of the present invention predicting the risk to suffer from infarction of the ischemic penumbra in a subject suffering from ischemic stroke, said kit comprising a detection agent for the biomarker sFlt-1 as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as an optical storage medium (e.g., a Compact Disc) or directly on a computer or data processing device. Moreover, the kit may, preferably, comprise standards for reference amounts as described elsewhere herein in detail.

The figures show:

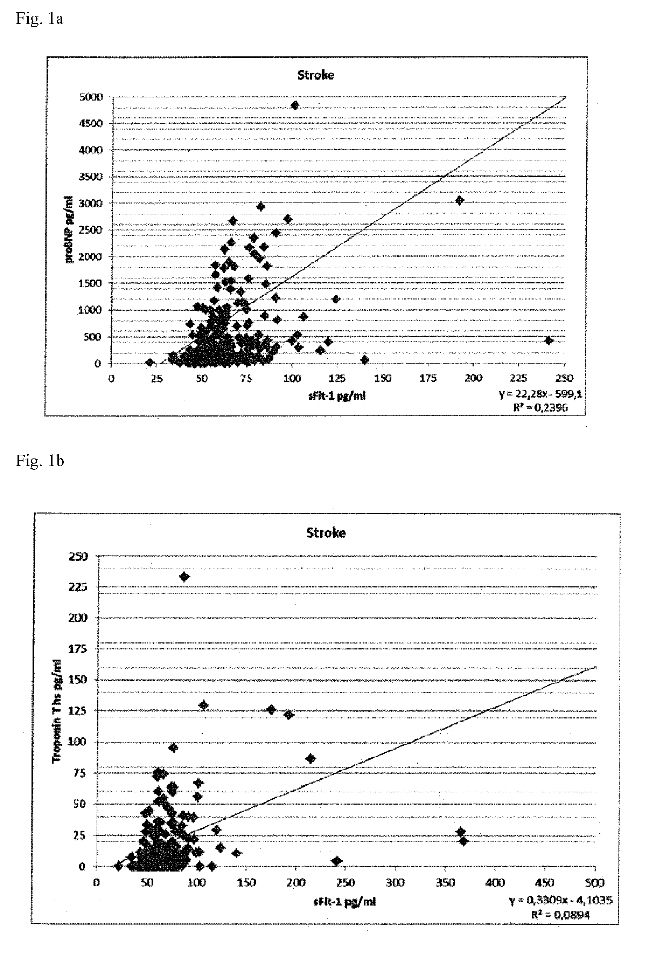

**Fig. 1a****:** Correlation of NT-proBNP to sFlt-1
**Fig. 2a:** Correlation of Troponin to sFlt-1

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

### Example 1: Patient cohorts

A total of 254 patients presenting with stroke (mean age 70 years) were included into the study. Ischemic stroke was confirmed by imaging (CT or MRI Scan). Samples were taken at presentation (between 2.5 and 34 hours after stroke onset, median 7.3 hours) as well as 6 and 24 hours after presentation. Patients with stroke were further classified into TIA (transitory ischemic attack), minor and major stroke.

In order to assess sFlt-11 in stable patients with heart failure, 42 patients with stable heart failure (change of body weight below 2 kg 4 weeks before entry into the study, no signs of symptoms compatible with chest pain or even ACS.) were tested at entry and 2 and 4 weeks thereafter.

Further, a total of 109 patients with stable heart failure (no signs of acute cardiovascular disease, no change in body weight of more than 2 kg in the previous two weeks) were used as a control population.

### Example 2: Determination ofsFlt-1, NT-proBNP, and of Troponin T

sFlt-1, Troponin T (hsTNT), and NT-proBNP were determined with sandwich immunoassays using analyzers from Elecsys or COBAS e-series. The assays comprise two monoclonal antibodies specific for the respective peptide. The first of these antibodies is biotinylated and the second one in labelled with a Tris(2,2'-bibyridyl)ruthenium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. sFlt-1 amounts between 10 to 85,000 pg/ml, GDF-15 amounts between 300 pg to 20,000 pg/ml, and NT-proBNP amounts between 2 pg/ml and 35,000 pg/ml can be measured. The high sensitivity Troponin T test used in this study has a sensitivity of 2 pg/ml. The amounts of sFlt-1, Troponin T and NT-proBNP in Examples are given in "pg/ml".

### Example 3: Results

### Results in 109 patients with stable heart failure

| sFlt-1 below median | sFlt-1 above Median | |
|---|---|---|
| 65,5 pg/ml | 65,5 pg/ml | |
| sFlt-1 | 53,2 (48,3/58,3) | 80,3 (72,1/96,0) |
| NT-pro BNP pg/ml | 547 (225/1193) | 1687 (505/2950) |
| Hs Troponin T pg/ml | 7,5 (3,3/15,8) | 19,8 (7,5/33,7) |
| | | |
| NYHA class | | |
| NYHA I | 28 | 17 |
| NYHA II | 15 | 17 |
| NYHA III | 9 | 19 |
| NYHA IV | 0 | 0 |

As can be seen from the above, in stable patients with stable heart failure sFlt-1 levels (above median) were associated with higher NT-pro BNP levels and markers of chronic necrosis (Troponin T). Also patients with higher sFlt-1 levels has lower blood pressure levels as an indicator of lower cardiac output and chronic ischemia.

### Results in 42 patients with stable heart failure

Results of the 42 stable patients with heart failure are shown in the Table below:

| | **sFlt-1** (pg/ml) | **NT-proBNP**(pg/ml) | **hs'TnT** (pg(ml) |
|---|---|---|---|
| Baseline | 77 (68 - 87) | 608 (322 - 1793) | 12 (7 - 20) |
| 2 weeks | 76 (65 - 81) | 582 (268 - 1439) | 11 (6 - 18) |
| 4 weeks | 74 (61 - 87) | 532 (259 - 1149) | 12 (6 - 18) |

As can be seen from the Table, sFlt-1 was stable in stable patients with heart failure so was troponin T and NT -pro BNP (the latter to a lesser extent). The increase sFlt-1 levels indicate cardiac ischemia.

The same markers were also followed in stroke patients and were measured at admission and 6 and 24 hours thereafter:

| | **sFlt-1** (pg/ml) | **NT-proBNP** (pg/ml) | **hsTnT** (pg(ml) |
|---|---|---|---|
| Baseline | 60 (52 - 73) | 268 (79 - 754) | 7 (0 - 15) |
| 6 hours | 57 (50 - 76) | 330 (109 - 1159) | 7 (0 - 19) |
| 24 hours | 66 (55 - 87) | 352 (111 - 1146) | 8 (0 - 18) |

As can be seen both sFlt-1 and troponin T were quite stable in stroke patients on follow up, NT-pro BNP tended to increase over time (possibly due to a cardiac response to stroke). Thus sFlt-1. troponin T and NT-pro BNP follows the same principle as found in stable patients with heart failure.

Patients were further separated in **sFlt-1 quartiles** and correlated with cardiac tests and other results.

| | **sFlt-1** | | | |
|---|---|---|---|---|
| | Q1 | Q2 | Q3 | Q4 |
| N= | 65 | 65 | 65 | 65 |
| sFtl-1 | 48 (44-50) | 56 (54-58) | 65(63-70) | 85 (78-100) |
| NT-proBNP | 83 (33-199) | 220 (78-623) | 385 (132-1054) | 531 (287-2171) |
| | | | | |
| hsTroponin T | 0 (0-7) | 5 (0-10) | 9 (3-17) | 15 (7-35) |
| TIA | 28 | 26 | 21 | 11 |
| Minor stroke | 17 | 14 | 14 | 16 |
| Major stroke | 21 | 24 | 30 | 33 |
| AF (cont.) | 0 | 7 | 14 | 20 |
| AF (intermit) | 2 | 7 | 9 | 10 |
| Cardioembolic | 6 | 12 | 24 | 25 |
| Large vessel | 17 | 15 | 5 | 9 |
| Small vessel | 9 | 8 | 8 | 7 |

The data in the table clearly indicate that increased sFlt-1 concentrations correlate to all markers of cardiac function (NT-pro BNP), necrosis (troponin T), and cardiac causes of stroke (cardioembolic stroke) as well as severity of stroke (TIA, major stroke). When sFlt-1 was correlated to NT-pro BNP and Troponin T, none of these markers was stringently correlated to sFlt-1 indicating that the ischemia marker sFlt-1 cannot be replaced by markers reflecting different cardiac entities.

### Case studies

### Patient 1:

A 67 year old male presents with minor stroke, his blood pressure is 140/90 mmHg, he has no history of heart failure and NT-pro BNP is 245 pg/ml, sFlt-1 is 61 pg/ml indicating an appropriate cardiac function and no evidence of chronic cardiac ischemia. Using ultrasound he is found to have a 70 % stenosis of the right arteria carotis (the site of the stroke with left sided partial paresis). Due to adequate cardiac perfusion and lack of stenosis of the left arteria carotis intera and open Aa vertebrales, an angioplasty is not performed (which has the risk of emoblization und enlargement of the cerebral necrosis and stroke worsening).

### Patient 2:

A 62 year old male presents with minor stroke. He has a blood pressure of 220/120 mmHg, his NT-pro BNP is 265 pg/ml, sFlt-1 is 60 pg/ml. Because of adequate cardiac perfusion his blood pressure was carefully lowered and symptoms improve.

### Patient 3:

A 72 year old female presents with stroke, her NT-pro BNP is 630 pg/ml, sFlt-1 is 89 pg/ml. She has a contralateral stenosis of the arteria carotis of 60 %, her blood pressure is 160 mmHg. She is decided to have not her blood pressure lowered and to have Endarterectomy to improve cerebral perfusion.

### Patient 4:

A 75 years old mal with stroke and a history of heart failure and without evidence of pulmonary edema and a blood pressure of 120/75 mmHg has an NT-proBNP of 760 pg/ml and sFlt-1 of 98 pg/ml and, thus, systemic cardiac ischemia. He has no stenosis of large vessels. It is decided to lower existing doses of diuretic therapy and close monitoring of the blood pressure, whole body weight und pulmonary status (development of pulmonary edema).

### Patient 5:

A 69 year old male patient with known heart failure NYHA class III and NT-pro BNP levels of 715 pg/ml six months ago and 795 pg/ml 3 months ago and respective sFlT1 concentrations of 81 and 83 pg/ml developed an ischemic stroke with left sided hemiparesis. His sFlT1 at presentation is 82 pg/ml and NT-pro BNP is 815 pg/ml, blood pressure is 110 mmHg systolic. His low dose loop diuretics are discontinued and he receives dopamine for 4 hours and his symptoms improve. There are no extracranial stenoses to be removed.

### Conclusions applying to the subject matter of the invention:

The diagnosis of chronic cardiac ischemia is important. Although the severity of stroke is largely dependent on the extent of obstruction and the size of the obstructed vessel and thus the size of the affected brain area, there is consensus, that if blood flow is completely obstructed brain death will occur within 4 - 10 minutes (which is the case in cardiac arrest), if the blood flow is below 16 - 18 ml/100g tissue, necrosis will take place in one hour and if the blood flow is below 20 ml/100 g tissue brain might survive several hours or days (W.S. Smith et al, Chapter 364 in Harrison Principles of Internal medicine, 17th Ed.). This is in fact a call to adapt cardiac function in stroke patients in order to avoid additional brain necrosis, in particular in order to avoid infarction of the ischemic penumbra.

Ischemic stroke is difficult to treat, within the first 3 to 5 hours lysis of the thrombus is able to save brain tissue and to avoid further brain necrosis. A further possibility is the interventional removal of clots or stenosis of the intra and extracranial arteries that might impair blood supply, this is however associated with significant complications (Arnaout O.M. et al, Techn Vasc. Interventional Radiol 2012: 15: 87 - 92). In addition the management of blood pressure remains controversial (Ntaios G. et al, Current Opinion in Neurology 2010: 23: 46 - 52). The association of stroke with chronic heart failure has been noted (Haeusler K.G. et al, Stroke 2011: 42: 2977 - 2982), however the impact on treatment in acute stroke remained unclear.

As shown herein, cardiac ischemia as reflected by sFlt-1 levels have impact on general and specifically brain perfusion, this has impact on treatment which includes:
a) modification of heart failure therapy (avoidance of blood pressure and intravasal volume lowering therapy depending on status of heart failure)
b) avoidance of blood pressure lowering therapy.
c) Adaptation of interventional strategies to reduced cardiac output and related ischemia (more liberal indication for interventional procedures).

In summary, the recognition of cardiac ischemia and its importance for stroke treatment offers new possibilities in the adaptation of stroke and heart failure treatment and in optimization of outcome in stroke. Thus, the determination of sFlt-1 allows to identify cardiac ischemia in stroke patients which triggers therapeutic measures directed to reduce brain necrosis in stroke patients.

## Claims

1. A method for diagnosing chronic cardiac ischemia in a subject suffering from ischemic stroke, said method comprising the steps of
a) determining the amount of soluble fms-like tyrosine kinase-1 (sFlt-1) in a sample from said subject, and
b) comparing the amount of sFlt-1 in said sample to a reference amount, whereby chronic cardiac ischemia is diagnosed.

2. The method of claim 1, wherein said sample has been obtained up to 72 hours after the onset of symptoms of ischemic stroke, in particular wherein the sample has been obtained up to 24 hours after the onset of ischemic stroke.

3. The method of claim 1, wherein said sample has been obtained more than 24 hours, but not later than 72 hours after the onset of symptoms of ischemic stroke.

4. The method according to any one of claims 1 to 3, wherein the subject does not suffer from an acute cardiac event, in particular, wherein the subject does not suffer from an acute cardiac event that caused or that is associated with the ischemic stroke.

5. The method of any one of claims 1 to 4, wherein the reference amount is derived from a subject or a group of subjects suffering from an ischemic stroke, said subject(s) suffering from chronic cardiac ischemia, wherein an essentially identical amount or an increased amount of sFlt-1 in the test sample as compared to reference amount is indicative for chronic cardiac ischemia, and/or wherein the reference amount is derived from a subject or a group of subjects suffering from an ischemic stroke, said subject(s) not suffering from chronic cardiac ischemia, wherein an essentially identical amount or an decreased amount of sFlt-1 in the test sample as compared to reference amount indicates that the subject does not suffer from chronic cardiac ischemia.

6. A method for predicting in a subject suffering from ischemic stroke the risk to suffer from infarction of the ischemic penumbra, the method comprising
a) determining the amount of soluble fms-like tyrosine kinase-1 (sFlt-1) in a sample from said subject, and
b) comparing the amount of sFlt-1 in said sample to a reference amount, whereby the risk to suffer from infarction of the ischemic penumbra is predicted.

7. A method for deciding whether a subject who suffers from ischemic stroke is susceptible to a therapy that lowers to blood pressure and/or to a therapy that increases cerebral perfusion, the method comprising the steps of
a) determining the amount of soluble fms-like tyrosine kinase-1 (sFlt-1) in a sample from said subject, and
b) comparing the amount of sFlt-1 in said sample to a reference amount, whereby is it decided whether the subject is susceptible to therapy that lowers to blood pressure and/or to a therapy that increases cerebral perfusion.

8. The method of claim 7, wherein the therapy that lowers the blood pressure is selected is administration of a blood pressure lowering drug, in particular a drug selected from the group consisting of ACE inhibitors, Beta adrenergic blockers, angiotensin-II receptor antagonists, and diuretics.

9. The method of claims 7 and 8, wherein the therapy is a therapy that lowers the blood pressure and, wherein i) the reference amount is derived from a subject or a group of subjects suffering from an ischemic stroke, wherein said subject(s) are known to be susceptible for a therapy for lowering the blood pressure, wherein an essentially identical amount or a decreased amount of sFlt-1 in the test sample as compared to reference amount is indicative for a subject who is susceptible to a therapy that lowers the blood pressure and/or wherein ii) the reference amount is derived from a subject or a group of subjects suffering from an ischemic stroke, wherein said subject(s) are known not to be susceptible to a therapy that lowers the blood pressure, wherein an essentially identical amount or an increased amount of sFlt-1 in the test sample as compared to reference amount is indicative for a subject who is not susceptible to a therapy that lowers the blood pressure.

10. The method of claim 9, wherein the therapy that increases cerebral perfusion is selected from a therapy that increases blood pressure, intravenous thrombolysis, in particular within three hours after the onset of symptoms of stroke, intra- and extracranial endovascular intervention, in particular stenting, antithrombotic treatment and anticoagulation.

11. The method of claim 10, wherein the therapy that increases blood pressure is administration of a blood pressure increasing drug, in particular of phenylephrine, midodrine, fludrocortisone, NaCl, dopamine, diaspirin, cross-linked hemoglobin, or low-molecular dextrans, and/or wherein the therapy that increases blood pressure is a discontinuation of administration of blood pressure lowering drugs.

12. The method of any one of claims 7, 10 and 11 , wherein the therapy is a therapy for increasing cerebral perfusion and, wherein i) the reference amount is derived from a subject or a group of subjects suffering from an ischemic stroke, wherein said subject(s) are known to be susceptible for a therapy for increasing cerebral perfusion, wherein an essentially identical amount or an increased amount of sFlt-1 in the test sample as compared to reference amount is indicative for a subject who is susceptible to a therapy for increasing cerebral perfusion and/or wherein ii) the reference amount is derived from a subject or a group of subjects suffering from an ischemic stroke, wherein said subject(s) are known not to be susceptible to a therapy for increasing cerebral perfusion, wherein an essentially identical amount or a decreased amount of sFlt-1 in the test sample as compared to reference amount is indicative for a subject who is not susceptible to a therapy for increasing cerebral perfusion.

13. The method of any one of claims 1 to 12, further comprising the determination of a the amount of a natriuretic peptide in a sample from the subject, and the comparison with a reference amount.

14. Use of the biomarker sFlt1, or of a detection agent which specifically binds thereto in a sample of a subject suffering from an ischemic stroke for diagnosing chronic cardiac ischemia in said subject, for predicting the risk of said subject to suffer from infarction of the ischemic penumbra, or for deciding whether said subject who suffers from ischemic stroke is susceptible to a therapy that lowers to blood pressure and/or to a therapy that increases cerebral perfusion.

15. A device adapted for carrying out the method of the present invention for diagnosing chronic cardiac ischemia in a subject suffering from ischemic stroke, comprising
a) an analyzing unit comprising a detection agent which specifically binds to sFlt1, said unit being adapted for determining the amount of sFlt-1 in a sample of a said subject; and
b) an evaluation unit for comparing the determined amount with a reference amount whereby chronic cardiac ischemia can be diagnosed, said unit comprising a database with reference amount values (a reference amount value) derived from a subject or group of subjects as defined in 5, and a computer-implemented algorithm for diagnosing chronic cardiac ischemia as set forth above.
